# DEMANDE DE BREVET EUROPEEN

(11) **EP 1 125 891 A1**
(43) Date de publication de la demande: **22.08.2001**
(21) Numéro de dépôt: 00200533.8
(22) Date de dépôt: 17.02.2000
(51) Int. Cl.: C01B 37/00, B01J 29/04, C07D 301/12

(54) **Procédé de fabrication d'un solide cristallin**

(71) Demandeur: SOLVAY (Société Anonyme), 1050 Bruxelles (BE)
(72) Inventeur: Schoebrechts, Jean-Paul, 1390 Grez-Doiceau (BE)
(74) Mandataire: Vande Gucht, Anne

(57) **Abrégé**

Procédé de fabrication de solides cristallins contenant au moins un élément choisi parmi les groupes IIIa, IVa et Va et au moins un métal de transition, selon lequel on hydrolyse au moins une source d'oxyde de l'élément des groupes IIIa, IVa et Va et au moins une source d'oxyde du métal de transition qui a été préalablement dissoute dans un alcool ayant un pKa inférieur à celui de l'eau, dans un milieu aqueux comprenant un agent minéralisant et on cristallise le gel ainsi obtenu en présence d'un agent structurant.

## Description

La présente invention concerne la fabrication de solides cristallins et en particulier, de zéolites au titane.

Les premières synthèses de zéolites au titane décrites dans la littérature font l'objet des brevets US 4666692 et 4410501, le dernier étant spécifique aux silicalites de titane. La méthode de synthèse utilisée consiste en un traitement hydrothermique d'un gel obtenu par réaction entre une source d'oxyde de silicium hydrolysable (tel qu'un alcoolate), un alcoxyde de titane, éventuellement un oxyde alcalin, l'hydroxyde de tétrapropylammonium qui joue à la fois le rôle d'agent minéralisant (qui donne les ions OH- nécessaires à l'hydrolyse) et d'agent structurant (qui influence la structure cristalline), et de l'eau. Lorsqu'on veut incorporer des quantités importantes de titane dans le silicalite, la source d'oxyde de titane, plus réactive que celle d'oxyde de silicium, doit être traitée au préalable par une solution d'H₂O₂ afin d'éviter la précipitation d'oxyde de titane sous forme d'anatase en dehors du réseau cristallin. Ce traitement et l'addition subséquente de la base organique azotée se font lentement et à froid, ce qui rend la procédure de fabrication laborieuse. En outre, avant cristallisation du silicalite, les alcools résultant de l'hydrolyse des alcoolates de silicium et de titane sont évaporés. Cette évaporation prend du temps, consomme de l'énergie et constitue par ailleurs une opération délicate à réaliser à l'échelle industrielle, qui nécessite en outre l'ajustement de la teneur en eau après évaporation.

Thangaraj et al. (Zeolites, 1992, Vol.12, November/December, p.943-950) ont proposé une procédure modifiée pour la synthèse de TS-1 permettant de remédier à l'utilisation de solutions d'H₂O₂ et d'incorporer de plus grandes quantités de titane dans le réseau. Cette méthode consiste à ajouter une première partie de la solution de la base organique azotée (TPAOH ou hydroxyde de tétra-n-propylammonium) à du TEOS (tétraéthoxyde de silicium) et à ajouter goutte à goutte à l'ensemble, du TBOT (tétra n-butoxide de titane) en solution dans de l'iPrOH (alcool isopropylique). Après agitation, on rajoute le restant de la solution de la base organique azotée et on chauffe pour évaporer l'alcool. Cette méthode présente l'inconvénient d'utiliser une solution alcoolique de TBOT qui est peu stable par rapport à l'hydrolyse et donc, favorise la formation de précipités d'oxyde de titane. En outre, elle inclut un stade d'évaporation des alcools présents dans le gel avant cristallisation.

Récemment, Tuel (Catalysis Letters 51, 1998, p. 59-63) a montré que le TS-1 peut être synthétisé au départ des mêmes réactifs sans évaporation des alcools isopropylique (iPrOH), éthylique (EtOH) et n-butylique (nBuOH) présents dans le gel et ceci sans influence sur la structure cristalline et l'activité catalytique du TS-1 lorsqu'il est utilisé comme catalyseur des réactions d'hydroxylation de phénols avec de l'H₂O₂. Cette méthode présente néanmoins toujours l'inconvénient d'utiliser une solution alcoolique de l'alcoolate de titane qui est peu stable par rapport à l'hydrolyse et qui doit donc être utilisée rapidement après sa fabrication et avec prudence.

La demanderesse a constaté que, de manière surprenante, lors de la synthèse de solides cristallins au départ d'au moins une source d'oxyde de l'élément des groupes IIIa, IVa et Va et d'au moins une source d'oxyde de métal de transition, le choix d'un alcool ayant un pKa inférieur à celui de l'eau pour dissoudre la source d'oxyde de métal de transition permet d'obtenir une solution stable qui ne mène pas à la formation d'un précipité lors de l'addition de cette solution au milieu aqueux d'hydrolyse et qui peut être stockée avant son utilisation. En outre, cet alcool peut rester présent lors de la cristallisation sans influencer la structure cristalline et l'activité catalytique du solide cristallin obtenu.

En conséquence, le fait d'utiliser un alcool ayant un pKa inférieur à celui de l'eau pour dissoudre la source de l'oxyde du métal de transition permet l'obtention des solides cristallins selon un procédé facile à mettre oeuvre, qui permet de réduire, voire d'éviter, aisément la formation de précipités d'oxydes métalliques en dehors du réseau cristallin, comme par exemple l'anatase.

La présente invention concerne dès lors un procédé de fabrication de solides cristallins contenant au moins un élément choisi parmi les groupes IIIa, IVa et Va et au moins un métal de transition, selon lequel on hydrolyse, dans un milieu aqueux comprenant un agent minéralisant, au moins une source d'oxyde de l'élément des groupes IIIa, IVa et Va et au moins une source d'oxyde du métal de transition qui a été préalablement dissoute dans un alcool ayant un pKa inférieur à celui de l'eau, et on cristallise le gel ainsi obtenu en présence d'un agent structurant.

Les solides cristallins selon la présente invention désignent tous les solides où les atomes sont ordonnés de manière à définir un réseau cristallin et qui contiennent au moins un élément choisi parmi les groupes IIIa, IVa et Va et au moins un métal de transition. L'élément des groupes IIIa, IVa et Va peut être l'aluminium, le silicium ou le phosphore. De préférence, cet élément est le silicium. Le métal de transition peut être le titane, le vanadium, le zirconium, le chrome, le fer ou le cobalt. De préférence, ce métal est le titane.

Ces solides cristallins sont avantageusement des zéolites. De manière préférée, ce sont des zéolites au titane. Par zéolite au titane, on entend désigner un solide contenant de la silice qui présente une structure cristalline microporeuse de type zéolite et dans laquelle plusieurs atomes de silicium sont remplacés par des atomes de titane. La zéolite au titane présente avantageusement une structure cristalline de type ZSM-5 ou ZSM-11. Elle peut aussi présenter une structure cristalline de type zéolite β exempte d'aluminium. Elle présente de préférence une bande d'absorption infrarouge à environ 950 - 960 cm⁻¹. Les zéolites au titane de type silicalite conviennent bien. Celles répondant à la formule xTiO₂(1-x)SiO₂ dans laquelle x est de 0,0001 à 0,5, de préférence de 0,001 à 0,05, sont performantes. Des matériaux de ce type, connus sous le nom de TS-1, présentent une structure zéolitique cristalline microporeuse analogue à celle de la zéolite ZSM-5. Les propriétés et les principales applications de ces composés sont connues (B. Notari; Structure-Activity and Selectivity Relationship in Heterogeneous Catalysis; R.K. Grasselli and A.W. Sleight Editors; Elsevier; 1991; p. 243-256).

La source d'oxyde de l'élément issu des groupes IIIa, IVa et Va est un composé hydrolysable de cet élément tel que par exemple les alcoolates de cet élément. Lorsqu'il s'agit du silicium, la source peut être choisie parmi la silice colloïdale, les silicates de métaux alcalins ou alcalino-terreux et les alcoolates de silicium. Ces derniers conviennent particulièrement bien.

La source d'oxyde de métal de transition selon l'invention peut être un halogénure de métal de transition, comme par exemple le TiCl₃ ou un alcoolate de métal de transition. Les alcoolates de métal de transition conviennent particulièrement bien. Les alcoolates de titane donnent de bons résultats.

Par alcoolates de silicium ou de titane, on entend désigner des composés répondant à la formule générale M(OR)₄ où M est soit le silicium, soit le titane et R, un groupement hydrocarboné. De préférence, l'alcoolate de silicium est un tétraalcoxyde de silicium. Le groupement alcoxyde comprend avantageusement jusqu'à 10 atomes de carbone, de préférence jusqu'à 6 atomes de carbone et plus particulièrement jusqu'à 4 atomes de carbone. Le tétraéthoxyde de silicium ou TEOS donne de bons résultats. De préférence, l'alcoolate de titane est un tétraalcoxyde de titane. Le groupement alcoxyde comprend avantageusement jusqu'à 10 atomes de carbone, de préférence jusqu'à 6 atomes de carbone, et plus particulièrement jusqu'à 4 atomes de carbone. Avec le procédé selon l'invention, de bons résultats sont obtenus lorsque l'alcoolate de titane est dérivé d'un alcool qui a un pKa supérieur à celui de l'eau. Des exemples de tels alcools sont l'éthanol, le propanol et le butanol. Le tétra-n-butoxyde de titane ou TBOT donne d'excellents résultats.

L'agent minéralisant, qui fournit les ions hydroxydes nécessaires à l'hydrolyse des sources d'oxyde de l'élément des groupes IIIa, IVa et Va et de l'oxyde de métal de transition, et l'agent structurant, qui favorise la cristallisation vers la structure cristalline adéquate, peuvent être deux composés séparés, tels que respectivement les bases de métaux alcalins ou alcalino-terreux d'une part, et les amines, les phosphines, les sels d'ammonium, les sels de phosphonium, les alcools ou les acides aminés d'autre part. Alternativement, il peut s'agir d'un seul et même composé tel qu'une base d'ammonium quaternaire. Les bases de tétraalkylammonium (l'alkyle pouvant contenir jusqu'à 10 atomes de carbone, en particulier jusqu'à 6 atomes de carbone, de préférence jusqu'à 4 atomes de carbone), telle que l'hydroxyde de tétra-n-propylammonium ou TPAOH, conviennent particulièrement bien pour jouer à la fois le rôle d'agent minéralisant et d'agent structurant. L'hydroxyde de tétraéthylammonium ou de tétra-n-butylammonium conviennent également bien. Parfois, il peut s'avérer économiquement intéressant de remplacer une partie de cette base par l'amine tertiaire correspondante. Ainsi, on peut mélanger la base avec l'amine en des quantités telles que le rapport molaire (amine)/(base + amine) soit de 1 à 35 %, en particulier de 20 à 30 %, par exemple environ 25 %. De bons résultats sont notamment obtenus en mélangeant du TPAOH avec de la tripropylamine dans ces proportions.

L'agent minéralisant est généralement en solution aqueuse à une concentration en poids supérieure ou égale à 10%, et de préférence, supérieure ou égale à 15%. La concentration en poids d'agent minéralisant est de préférence inférieure ou égale à 50%, voire même inférieure ou égale à 25%. Une concentration d'environ 20% donne de bons résultats.

La solution d'agent minéralisant est généralement pauvre en métaux alcalins et alcalino-terreux. De préférence, elle contient moins de 200 ppm, voire même moins de 25 ppm de ces métaux et idéalement, moins de 2 ppm.

Dans le cadre de l'invention, il convient de conférer au terme « alcool », une acceptation tout à fait générale et y inclure les phénols (non substitués et substitués partiellement ou totalement), aussi bien que les alcools aliphatiques linéaires, cycliques, saturés, insaturés, non substitués et substitués partiellement ou totalement. Les alcools aliphatiques sont particulièrement recommandés. Ceux qui contiennent jusqu'à 10 atomes de carbone conviennent bien, en particulier jusqu'à 6 atomes de carbone, par exemple jusqu'à 4 atomes de carbone. Des exemples d'alcools aliphatiques ayant un pKa inférieur à celui de l'eau conformément à l'invention, sont le 2,2,2-trifluoroéthanol, le 2-méthoxyéthanol, et le 2-éthoxyéthanol (EtOEtOH). Ce dernier donne de bons résultats.

De même, dans le cadre de cette invention, il convient de conférer au terme « gel », une acceptation large qui va de la simple solution (fluide) à un vrai gel de forte consistance.

Il peut être avantageux de chauffer la solution obtenue par dissolution de la source d'oxyde de métal de transition dans l'alcool ayant un pKa inférieur à celui de l'eau. A cet effet, cette solution est généralement chauffée à une température d'au moins 30 °C, en particulier d'au moins 32 °C. La température ne dépasse habituellement pas 50 °C, en particulier pas 40 °C, une température voisine de 35 °C donne de bons résultats. La durée pendant laquelle la solution peut être chauffée est couramment d'au moins 1 min, en particulier d'au moins 10 min. La durée ne dépasse en général pas 60 min, en particulier pas 45 min. Une durée voisine de 30 min donne de bons résultats.

Les réactifs participant à la formation du gel à cristalliser, c.à.d. la source d'oxyde de l'élément issu des groupes IIIa, IVa et Va, la source d'oxyde de métal de transition en solution alcoolique, l'eau et l'agent minéralisant peuvent être mélangés dans un ordre arbitraire, en prenant soin toutefois de ne pas former de précipité d'oxyde de métal de transition.

Une méthode possible consiste à mélanger d'abord la source d'oxyde de l'élément issu des groupes IIIa, IVa et Va, la source d'oxyde de métal de transition et l'alcool, et à ajouter ensuite lentement et de préférence à froid (de 0 à 5 °C), une solution aqueuse de l'agent minéralisant.

Selon une autre méthode, on prépare une première solution par mélange de la source d'oxyde de l'élément issu des groupes IIIa, IVa et Va avec une première fraction d'une solution aqueuse de l'agent minéralisant; on prépare une deuxième solution par mélange de la source d'oxyde de métal de transition avec l'alcool ; on mélange ces deux solutions et on ajoute une deuxième fraction de la solution de l'agent minéralisant à ce mélange. Dans cette méthode, la fraction de la solution de l'agent minéralisant ajoutée à la source d'oxyde de l'élément issu des groupes IIIa, IVa et Va est généralement supérieure à 60% de la fraction totale d'agent minéralisant, de préférence supérieure à 70%. Elle ne dépasse avantageusement pas 99%, voire 90%. Cette seconde méthode présente deux avantages par rapport à la première: d'une part, elle permet de réduire la proportion de métal de transition qui n'est pas introduite dans le réseau cristallin, et d'autre part, elle permet de réaliser l'opération d'hydrolyse à température ambiante et non à froid, et en un temps plus court.

Selon encore une autre méthode, on prépare d'abord une première solution en mélangeant la source d'oxyde de l'élément issu des groupes IIIa, IVa et Va avec la totalité de l'agent minéralisant, ensuite on prépare une deuxième solution par mélange de la source d'oxyde de métal de transition avec l'alcool et on mélange les deux solutions.

La durée, c.à.d. la vitesse, de ces opérations de mélange et d'agitation sont adaptées de manière à éviter la formation d'un précipité.

De préférence, les mélanges susmentionnés se font sous azote. Ils peuvent être suivis d'une évaporation des alcools par chauffage à une température d'au moins 60°C, voire d'au moins 75°C. Cette température est généralement inférieure ou égale à 100°C, de préférence inférieure ou égale à 90°C. Une température voisine de 85 °C convient bien. La durée du chauffage est de préférence celle nécessaire à l'élimination totale des alcools. Elle peut varier entre 1h et 10h, en particulier entre 3 et 5 h.

Les gels exempts ou non d'alcools peuvent ensuite être additionnés d'eau et/ou d'alcool afin d'ajuster leur composition avant cristallisation.

De manière surprenante, le fait de réaliser la cristallisation en présence d'alcool(s) permet d'augmenter le rendement en solides de la réaction (c.à.d. la masse de solide récupérée après calcination, rapportée à sa valeur théorique calculée sur base des masses de précurseurs d'oxyde de l'élément issu des groupes IIIa, Iva et Va et de métal de transition mis en oeuvre).

La composition du gel avant cristallisation présente généralement:
- un rapport molaire (métal de transition/élément issu des groupes IIIa, IVa et Va) généralement supérieur ou égal à 0,0005 mol/mol, et de préférence supérieur ou égal à 0,001 mol/mol; ce rapport molaire n'excède généralement pas 0,1 mol/mol et de préférence, pas 0,05 mol/mol;
- un rapport molaire (agent minéralisant et structurant/élément issu des groupes IIIa, IVa et Va) généralement supérieur ou égal à 0,05 mol/mol, et de préférence supérieur ou égal à 0,1 mol/mol; ce rapport molaire n'excède généralement pas 1 mol/mol et de préférence, pas 0,5 mol/mol; les valeurs de 0,12 à 0,3 étant courantes.
- un rapport molaire (H₂O/élément issu des groupes IIIa, IVa et Va) généralement supérieur ou égal à 5 mol/mol, et de préférence supérieur ou égal à 10 mol/mol; ce rapport molaire n'excède généralement pas 200 mol/mol et de préférence, pas 100 mol/mol; les valeurs de 10 à 40 étant courantes.
- un rapport molaire (alcool issu de l'hydrolyse de la source d'oxyde de l'élément issu des groupes IIIa, IVa et Va/élément issu des groupes IIIa, IVa et Va) généralement supérieur ou égal à 0 mol/mol; ce rapport molaire n'excède généralement pas 4 mol/mol;
- un rapport molaire (alcool issu de l'hydrolyse de la source d'oxyde de métal de transition/élément issu des groupes IIIa, IVa et Va) généralement supérieur ou égal à 0 mol/mol; ce rapport molaire n'excède généralement pas 0,2 mol/mol;
- un rapport molaire (alcool qui dissout la source d'oxyde de métal de transition/ élément issu des groupes IIIa, IVa et Va) généralement supérieur ou égal à 0,002 mol/mol, et de préférence supérieur ou égal à 0,1 mol/mol; ce rapport molaire n'excède généralement pas 10 mol/mol et de préférence, pas 8 mol/mol; les valeurs de 0,75 à 5 étant courantes.

Généralement, le gel à cristalliser est à un pH alcalin, et de préférence au moins égal à 10.

La cristallisation s'opère généralement au cours d'un traitement thermique, qui peut se dérouler en autoclave.

On appelle autoclave, un récipient hermétiquement fermé bénéficiant de la résistance mécanique et chimique adéquate pour pouvoir résister à la pression, à la température et aux réactifs présents en son sein. Ce récipient est en outre pourvu d'un ou de plusieurs moyens de conditionnement thermique et d'agitation. Il est également pourvu de moyens de régulation en température et en pression, ainsi que de dispositifs de sécurité adéquats.

La durée du traitement thermique est de préférence supérieure ou égale à 1 h, voire même supérieure ou égale à 5 h. Préférablement, la durée du traitement thermique n'excède pas 120 h, voire même 72 h, les durées de 10 à 24 h étant courantes.

La température moyenne durant le traitement thermique est généralement supérieure ou égale à 100 °C, de préférence supérieure ou égale à 130 °C. Elle est généralement inférieure ou égale à 220 °C, de préférence inférieure ou égale à 200 °C, les températures de 150 à 180 °C étant courantes, par exemple environ 175 °C.

Après cristallisation, les particules solides en suspension peuvent être séparées du milieu réactionnel liquide par des moyens classiques (centrifugation, filtration, atomisation ...) et éventuellement lavées.

Les particules cristallisées, séparées ou non du milieu réactionnel liquide, sont préférablement séchées. Le séchage peut s'effectuer de toute manière connue.

Les particules cristallisées, séchées ou non, sont généralement soumises à calcination, de préférence sous air. La durée de calcination est généralement supérieure ou égale à 1 h, de préférence supérieure ou égale à 2 h. La durée de calcination est généralement inférieure ou égale à 72 h, de préférence inférieure ou égale à 24 h, les durées de 5 à 15 h étant courantes. La température de calcination est généralement supérieure ou égale à 400 °C, de préférence supérieure ou égale à 500 °C. La température de calcination est généralement inférieure ou égale à 650 °C, de préférence inférieure ou égale à 600 °C. Elle est par exemple environ 550 °C.

Le procédé selon l'invention peut avantageusement être intégré dans un procédé de fabrication d'un oxiranne par réaction entre un composé oléfinique, de préférence le chlorure d'allyle ou le propylène, et un composé peroxydé, de préférence le peroxyde d'hydrogène.

Le procédé selon l'invention concerne dès lors également un procédé de fabrication d'un oxiranne dans lequel on fabrique un solide cristallin selon la méthode décrite ci-dessus et on utilise ce solide cristallin comme catalyseur de la réaction entre un composé oléfinique, de préférence le chlorure d'allyle ou le propylène, et un composé peroxydé, de préférence le peroxyde d'hydrogène.

La présente invention est illustrée de manière non limitative par les exemples suivants. Les conditions expérimentales relatives à ces exemple sont décrites ci-dessous et les résultats figurent dans le tableau 1.

### Exemple C1 : non conforme à l'invention

On a mélangé à l'abri de l'air 35 g de TEOS (environ 38 ml) et 0.96 g (0.89 ml) de TEOT, et on a agité le mélange à 35°C, sous azote, pendant 30 min.

Ensuite, on a refroidi le mélange à 0 °C sous azote et sous agitation, pour y ajouter 47.5 g (47.5 ml) d'une solution aqueuse de TPAOH à 20% en poids selon le profil de vitesse d'addition suivant: 0.1 ml/min pour les 2 premiers ml, 0.2 ml/min pour les 2 suivants, 0.5 ml/min pour les 4 suivants et 1.0 ml/min pour le solde.

On a observé l'apparition d'un précipité lors de l'ajout des 2 premiers ml, d'une floculation entre 2 et 4 ml et d'une redissolution entre 4 et 47.5 ml.

La solution obtenue a été portée à 65°C en 0.5 h, et y est restée sous azote et sous agitation durant 3h.

Une quantité d'eau égale à 67 ml environ a été ajoutée. L'ensemble a été chauffé en autoclave à 175°C durant 64 h, puis filtré sous une pression de 7 bar sur filtre millipore 0.025 µm.

Le solide recueilli a été séché en étuve à 90°C durant une nuit, puis calciné sous air durant 10 h à 550°C. On a alors calculé le rendement en solide de la réaction.

Une fraction du silicalite obtenu a été analysée par ICP-OAS en vue de déterminer sa teneur en titane, et par XRD (diffraction X) en vue de déterminer sa teneur en anatase.

Une autre fraction du silicalite obtenu a été utilisée dans un test d'époxydation du CAL (chlorure d'allyle) par de l'H₂O₂ dilué (30-35% en poids), dans les conditions suivantes: solvant = CH₃OH, 25°C, 750 rpm, 2.6 mol CAL/kg, 1.3 mol H₂O₂/kg, CAL/ H₂O₂= 2 mol/mol, CH₃OH/CAL = 7.2 mol/mol, 2% en poids de catalyseur à 20 g Ti/kg. L' H₂O₂est ajouté en 20 min. de façon à limiter l'augmentation de la température du milieu réactionnel. Des prélèvements sont réalisés à 21 et 90 min. de réaction. Ils sont analysés par iodométrie pour la détermination de la teneur résiduelle en H₂O₂ et par chromatographie en phase gazeuse pour la détermination des composés organiques (uniquement la fraction recueillie en fin de réaction, soit à 90 min.).

### Exemple 2 : conforme à l'invention

La procédure suivie est identique à celle décrite ci-dessus pour l'exemple 1, sauf en ce qui concerne les points suivants:
- de l'EtOEtOH (0.1 mol/mol par rapport au Si) a été ajouté au mélange de TEOS et TEOT
- l'évaporation des alcools a été réalisée à 85°C
- la récupération du solide après traitement thermique a été effectuée par ultracentrifugation.

On n'a pas observé la formation d'un précipité lors de l'adjonction du TPAOH au mélange de TEOS, TEOT et EtOEtOH.

### Exemple C3 : non conforme à l'invention

La procédure qui a été suivie est conforme à celle recommandée par Thangaraj et comporte les étapes suivantes:
- dissolution sous azote du TBOT (1.86 g) dans de l'isopropanol (iPrOH) sec (13,3 ml) et agitation à 35°C pendant 30 min
- addition au TEOS (47,7 g) d'une partie de la solution aqueuse à 20 % en poids de TPAOH (48,2 ml ajoutés en 10 min à 25°C) et agitation pendant 15 min
- addition sous azote et à température ambiante de la solution de TBOT au TEOS préhydrolysé (16 ml/h) et maintien 30 min à cette température
- ajout du complément de TPAOH (16 ml à 16 ml/h) au mélange précédent
- la suite des opérations est identique à celle de l'exemple 2 (évaporation des alcools, traitement thermique, calcination et analyses).

On observe un léger précipité à l'extrémité du tube d'injection de la solution de TBOT dans l'iPrOH.

### Exemple 4 : conforme à l'invention

La procédure suivie est identique à celle décrite ci-dessus pour l'exemple 3, sauf que de le 2-éthoxyéthanol (EtOETOH) a été utilisé à la place de l'iPrOH, et que la durée de cristallisation était de 136 h.

On n'observe pas de précipité à l'extrémité du tube d'injection de la solution de TBOT dans l'EtOETOH.

### Exemple 5 : conforme à l'invention

La procédure suivie est identique à celle décrite ci-dessus pour l'exemple 3, sauf en ce qui concerne les points suivants:
- les quantités de réactifs utilisées mènent à un gel de composition molaire 1 Si - 0.034 Ti - 0.28 TPAOH - 28 H₂O-4,9 alcool;
- la nature de l'alcool: EtOEtOH;
- le fait que les alcools n'ont pas été évaporés avant la cristallisation;
- la durée de la cristallisation: 18 h;
- le fait que la récupération des solides a été effectuée par atomisation.

Les solutions de TBOT dans cet alcool sont stables à l'air pendant plusieurs jours et donc plus facilement manipulables que les solutions de TBOT dans l'iPrOH.

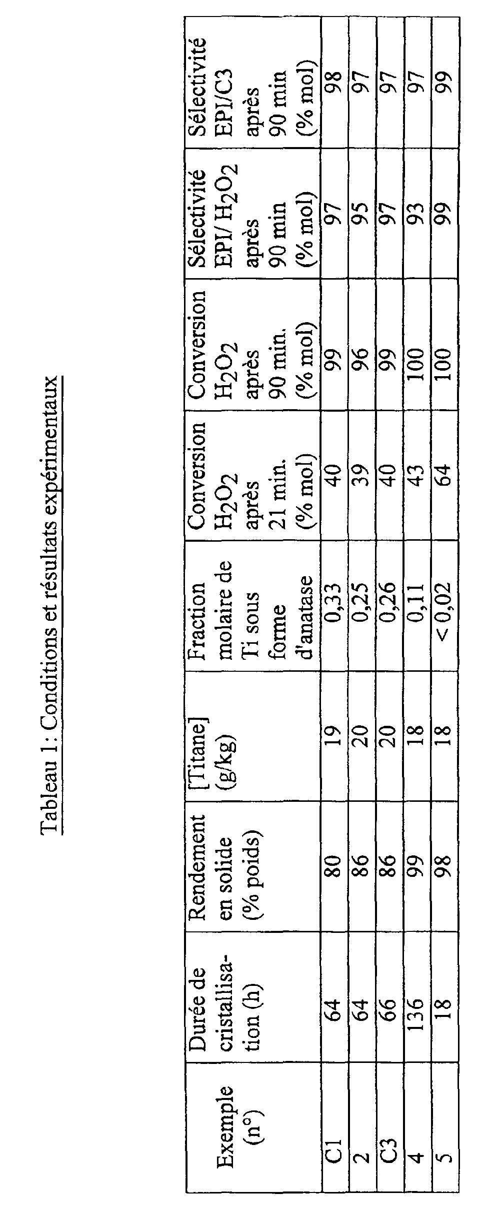

## Revendications

1. Procédé de fabrication de solides cristallins contenant au moins un élément choisi parmi les groupes IIIa, IVa et Va et au moins un métal de transition, selon lequel on hydrolyse, dans un milieu aqueux comprenant un agent minéralisant, au moins une source d'oxyde de l'élément des groupes IIIa, IVa et Va et au moins une source d'oxyde du métal de transition qui a été préalablement dissoute dans un alcool ayant un pKa inférieur à celui de l'eau, et on cristallise le gel ainsi obtenu en présence d'un agent structurant.

2. Procédé selon la revendication 1, dans lequel l'élément des groupes IIIa, IVa et Va est le silicium et le métal de transition est le titane.

3. Procédé selon la revendication 2, dans lequel le solide cristallin est une zéolite au titane, de préférence le TS-1.

4. Procédé selon la revendication 2 ou 3, dans lequel la source d'oxyde de silicium est un alcoolate de silicium, de préférence le tétraéthoxyde de silicium.

5. Procédé selon l'une quelconque des revendications 2 à 4, dans lequel la source d'oxyde de titane est un alcoolate de titane, de préférence un alcoolate de titane dérivé d'un alcool ayant un pKa supérieur à celui de l'eau, tel que le tétra-n-butoxyde de titane, et dans lequel l'alcool est le 2-éthoxyéthanol.

6. Procédé selon l'une quelconque des revendications précédentes, dans lequel l'hydroxyde de tétraalkylammonium, de préférence l'hydroxyde de tétra-n-propylammonium, joue à la fois le rôle d'agent minéralisant et d'agent structurant.

7. Procédé selon la revendication 6, dans lequel l'hydroxyde de tétraalkylammonium est introduit dans le milieu aqueux mélangé avec l'amine tertiaire correspondante.

8. Procédé selon l'une quelconque des revendications précédentes, dans lequel on prépare une première solution par mélange de la source d'oxyde de l'élément issu des groupes IIIa, IVa et Va avec une première fraction d'une solution aqueuse de l'agent minéralisant; on prépare une deuxième solution par mélange de la source d'oxyde de métal de transition avec l'alcool ; on mélange ces deux solutions et on ajoute une deuxième fraction de la solution de l'agent minéralisant à ce mélange.

9. Procédé selon l'une quelconque des revendications précédentes, dans lequel le gel n'est pas soumis à une évaporation des alcools avant cristallisation.

10. Procédé de fabrication d'un oxiranne, dans lequel on fabrique un solide cristallin selon la méthode décrite dans l'une quelconque des revendications précédentes et on utilise ce solide cristallin comme catalyseur de la réaction entre un composé oléfinique, de préférence le chlorure d'allyle ou le propylène, et un composé peroxydé, de préférence le peroxyde d'hydrogène.
